(19) Europäisches Patentamt
European Patent Office
Office européen des brevets

(11) Veröffentlichungsnummer: **0 129 636**
**B1**

(12) # EUROPÄISCHE PATENTSCHRIFT

(45) Veröffentlichungstag der Patentschrift: **04.01.89**

(51) Int. Cl.⁴: **C 07 D 311/58**, C 07 D 311/64, C 07 D 311/70, C 07 D 311/22, C 07 D 493/04, A 01 N 43/16

(21) Anmeldenummer: **84101503.5**

(22) Anmeldetag: **14.02.84**

(54) **Neues Verfahren zur Herstellung von Chromen-Derivaten.**

(30) Priorität: **16.02.83 HU 52083**

(43) Veröffentlichungstag der Anmeldung: **02.01.85 Patentblatt 85/1**

(45) Bekanntmachung des Hinweises auf die Patenterteilung: **04.01.89 Patentblatt 89/1**

(84) Benannte Vertragsstaaten: **AT BE CH DE FR GB IT LI NL SE**

(56) Entgegenhaltungen:
**EP-A-0 118 794**
**DE-A-2 639 671**
**DE-A-2 833 067**
**GB-A-1 357 633**
**US-A-4 162 326**

**TETRAHEDRON LETTERS, Band 21, 1980, Seiten 2361-2364, Pergamon Press Ltd., Oxford, GB., F. CAMPS et al.: "Fluorinated chromenes: 2,2,2-trifluoroethoxy precocene analogs and their corresponding 3,4-epoxides"**
**Römpps Chemie-Lexikon, Neumüller. Franck'sche Verlagshandlung (1973), pp. 38,126,2813**
**Monographie Chromenes, Chromanones and Chromones, G.P. ELLIS, John Wiley and Sons, N.Y. 1977, Seiten224-267**

(73) Patentinhaber: **ALKALOIDA VEGYéSZETI GYáR, Postfach 1, H-4440 Tiszavasvári (HU)**

(72) Erfinder: **Timár, Tibor, Dr. Dipl.- Chem., Elmunkás u. 27, H-4440 Tiszavasvári (HU)**
Erfinder: **Zsupán, Kálmán, Dr. Dipl.- Chem., Elmunkás u. 23/a, H-4440 Tiszavasvári (HU)**
Erfinder: **Répási, János, Dr., Elmunkás u. 1, H-4440 Tiszavasvári (HU)**
Erfinder: **Borsos, geb. Safranek, Irén, Elmunkás u. 21, H-4440 Tiszavasvári (HU)**
Erfinder: **Kiss, István, Dr., Zöldfa u. 4, H-6723 Szeged (HU)**
Erfinder: **Fodor, András, Dr., Retek u. 2, H-6723 Szeged (HU)**
Erfinder: **Maroy, Péter, Dr., Ürhajos u. 5/a, H-6723 Szeged (HU)**

(74) Vertreter: **Patentanwälte Beetz sen. - Beetz jun. Timpe - Siegfried - Schmitt- Fumian, Steinsdorfstrasse 10, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 129 636 B1

LIBER, STOCKHOLM 1989

**Beschreibung**

Verfahren und Zwischenprodukte zur Herstellung von Chromenderivaten und ihre Verwendung in Schädlingsbekämpfungsmitteln.

Die Erfindung betrifft ein Verfahren zur Herstellung neuer 2H-Chromenderivate, ihre Verwendung als Wirkstoffe in umweltfreundlichen Schädlingsbekämpfungsmitteln sowie entsprechende Zwischenprodukte. Die Verbindung sind Analoge der aus der Natur isolierten Verbindungen 7-Methoxy-2,2-dimethyl-2H-chromen (PRECOCEN 1, PI) und 6,7-Dimethoxy-2,2-dimethyl-2H-chromen (PRECOCEN 2, P2).

Die von P1 und P2 sowie ihre Antijuvenilhormonwirkung, die sterilisierende und eiabtötende Wirkung, sowie die Induktion des Ruhezustandes durch P1 und P2 bei Insekten und ihre Verwendbarkeit als umweltfreundliche Schädlingsbekämfungsmittel waren bereits bekannt (Bowers, W.S., Ohta, T., Cleere, J.H., Marsella, P.A., Science 193 (1976) 542; Fagoonee, I., Umrit, G., Insect. Sci. Its Appl. 1 (4) (1981) 373).

Über die Aktivität, die Wirkungsweise und den Metabolismus der Precocene sind zahlreiche Mitteilungen erschienen. Diesen gemäß wirken die Verbindungen durch die spezielle Schädigung der "corpora allata", des juvenilhormonproduzierenden Organs der Insekten. Nachdem die Wirkungen von P1 und P2 erkannt worden waren, wurde systematisch erforscht, in welcher Weise die bekannten biologischen Wirkungen dem 2H-Chromengerüst zugeordnet werden können. Zur Feststellung der Zusammenhänge zwischen Struktur und Wirkung sowie zur Herstellung der wirksamsten Analoga wurden ausgedehnte Forschungen betrieben. Nach den bisherigen Ergebnissen hängen Art und Ausmaß der festgestellten Wirkungen der Precocene stark von dem jeweiligen Schädling und von der Testmethode sowie aus chemischer Sicht von Anzahl, Art und Stellung der Substituenten des aromatischen Ringes, von der Stärke der Doppelbildung im Heteroring sowie von den elektronischen und sterischen Parametern des gesamten Moleküls ab (Bowers, W.S., Martinez-Paro, R., Science 197 (1977) 1369; Schooneveld, H., Experientia 35 (1979) 363; Bowers, W.S., Pontif. Acad. Sci. Scr. Varia 41 (1976) 129; Brooks, G.T. et al., Nature 281 (1979) 570; Ohta, T., Kagaku to Seibutsu 17 (2) (1979) 92; Ohta, T., Konchu no Seiri to Kagagu 63 (1979); Matolcsy et al., Z. Naturforsch. 35b (1980) 1449; Brocks, G.T. et al., Proc. Br. Crop. Prot. Conf, Pests. Dis. 1 (1979) 273; Pratt, G.E. et al., Nature 284 (1980) 320; Soderlund, D.M. et al., J. Agr. Food Chem. 28 (4) (1980) 724; Schooley, D.A. et al., Pestic. Biochem. Physiol. 13 (2) (1980) 95; Ottridge, A. P. et al., Pestic. Sci. 12 (3) (1981) 245.

Im Laufe dieser Forschungen wurden mehrere Derivate gefunden, deren Aktivität größer war als die der natürlichen Precocene, zum Beispiel 6-methyxy-7-ethoxy-2,2-dimethyl-2H-chromen(Precocen 3, P3, ZR 3623), das zehnmal so wirksam ist wie P2.

In der erschienenen Literatur und den Patentschriften sind insgesamt nur etwa 80 - 100 Analoga des Precocene angegeben, obwohl der Zusammenhang zwischen Struktur und Wirkung bekannt war. Der Grund hierfür liegt wahrscheilich in der Kompliziertheit der bisher bekanntgewordenen Synthesen.

Gemäß der Fachliteratur (CHROMENES, CHROMANONES and CHROMONES, Edited by G.P. Ellis, London, John, Wiley and Sons, (1977), S. 43 - 63) (1977) bieten sich zur Herstellung von 2H-Chromenen zahlreiche, voneindander unabhängige Möglichkeiten an. Die meisten von ihnen werden auch zur Herstellung von P1 und P2 sowie ihrer Analoga angewandt (Bowers, W.S., Ohta, T., Chem. Pharm. Bull. 25 (9) (1977) 2788; Gale, D.J., Wilshire, J.P.K., J. Text. Inst. 12 (1979) 525; Biernacki, W., Sobotka, W., Polih J. Chem. 53 (1979) 2275; 54 (1980) 2239; Cardillio, G. et al., Tetr. lett. 27 (1979) 2545; J. Chem. Soc. Chem. Comm. 836 (1979); Champs, F. et al., Tetr. Lett. 40 (1979) 3901; 21 (1980) 2361; J. Het. Chem. 17 (1980) 207 u. 1377; Tsukayama et al., Heterocycles 16 (6) (1981) 955, Banerji, A., Goomer, N.C., Ind. J. Chem 20B (1981) 144.

Aus GB-A-1 357 633, DE-A-2 639 671 und EP-A-1 118 794 sind Verfarhen zur Herstellung von 2H-Chromenderivaten bekannt, die sämtlich darauf beruhen, daß bei bereits vorgegebenen Substituenten am aromatischen Ring der heterocyclische Ring des Chromengerüsts in verschiedener Weise aufgebaut wird.

US-A-4 162 326 betrifft ferner ein Verfahren zur Herstellung von Chromenderivaten, bei dem ein Monohydroxychromanon, bei dem sich die Hydroxygruppen in 6- oder 7-Stellung befindet, mit einem Alkylierungsmittel alkyliert wird, woran sich eine Reduktion zum entsprechenden Chromanol sowie eine Dehydratisierung zum Chromenderivat anschließt.

Den gleichen Themenkreis betreffen die japanischen Patentschriften 73 121/79, 15 411/80, 40 637/80 und 43 039/80.

Den Patenschriften und sonstigen Publikationen ist gemeinsam, daß (obwohl unterschiedliche Synthesemethoden eingesetzt werden) die Herstellung jedes einzelnen Analogons ein individuell für die jeweilige Ausgangsverbindung zu lösendes Problem darstellt.

So ist zum Beispiel gemäß den bereits ausgeführten japansichen Patentschriften 15 411/80 und 40 637/80 sowie der DE-PS-2 639 671 zur Herstellung von P2 3,4-Dimethoxy-phenol erforderlich, während die Gewinnung des zehnmal aktiveren P3 von 3-Ethoxy-4-methoxyphenol ausgeht, dessen Herstellung in entsprechender Menge und Reinheit bereits an sich ein kompliziertes Problem ist.

Der Erfindung liegt die Aufbage zugrunde, ein neuartiges Verfahren zur Herstellung 0-disubstituierter 2H-Chromene, und entsprechende Zwischenprodukte anzugeben. Die Aufgabe wird gemäß dem kennzeichnenden Teil des Anspruchs 1 sowie gemäß den Anprüchen 7 und 8 gelöst.

Vorteilhafte Ausführungsformen des Verfahrens sind Gegenstand der abhängigen Ansprüche.

Das erfindungsgemäße Verfahren zur Herstellung von Chromenderivaten der allgemeinen Formel V,

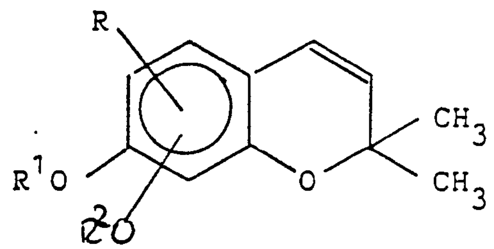

(V),

worin bedeuten:
R Wasserstoff oder $C_{1-8}$-Alkyl und
$R^1$ und $R^2$ $C_{1-10}$-Alkyl, Benzyl, Trifluorethyl oder Acetyl,
wobei $R^1$ und $R^2$ verschieden sind, oder zusammen eine Gruppe $-(CH_2)_3-$,
ist gekennzeichnet durch
(a1) Umsetzung eines Chromanonderivats der allgemeinen Formel I

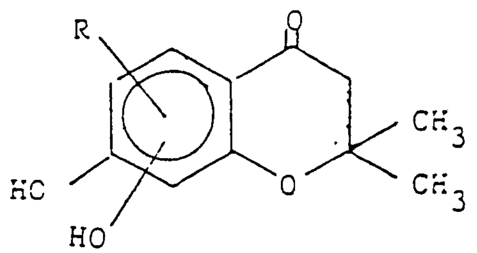

(I)

mit R wie oben
mit einem Reagens der allgemeinen Formel

$R^1$-X,

worin
$R^1$ dasselbe wie oben und
X den Reste eines Alkylierungsmittels oder Acetylierungsmittels bedeuten, in einer etwa äquivalenten Menge zu einem mono-O-substituierten Chromanonderivat der allgemeinen Formel II

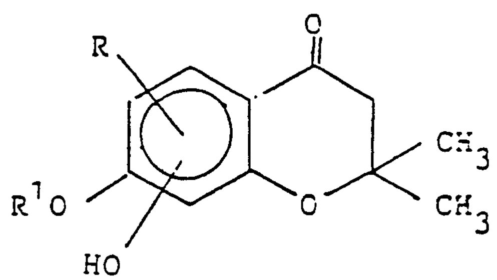

(II)

mit R und $R^1$ wie oben
und
Umsetzung des Chromanonderivats der Formel II mit einem Reagens der allgemeinen Formel

$R^2$-X

mit $R^2$ und X wie oben in einer Menge von 1,1 bis 1,5 mol pro Mol des Chromanonderivats der Formel II zu einem di-O-substituierten Chromanonderivat der allgeminen Formel III

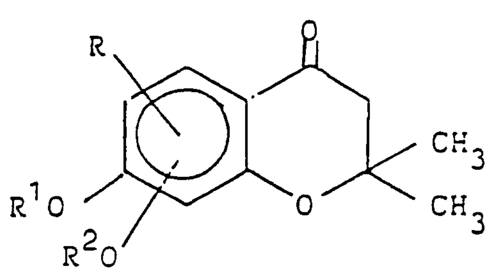

(III)

mit R, $R^1$ und $R^2$ wie oben
oder alternativ

(a2) direkte Umsetzung des Chromanonderivats der Formel I mit 1,3-Dibrompropan zum Chromanonderivat der Formel III, in der $R^1$ und $R^2$ zusammen $-(CH_2)_3-$ bedeuten,

(b) an sich bekannte Reduktion des Chromanonderivats der Formel III zum entsprechenden Chromanol der allgemienen Formel IV

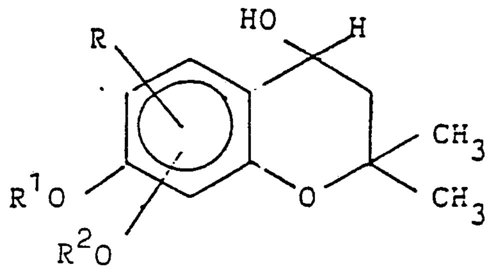

(IV)

mit R, $R^1$ und $R^2$ wie oben
und

(c) an sich bekannte Dehydratisierung des Chromanols der Formel IV in einem wäßrigen, sauren Medium.

Das erfindungsgemäße Verfahren ist im folgenden schematisch formelmäßig dargestellt.

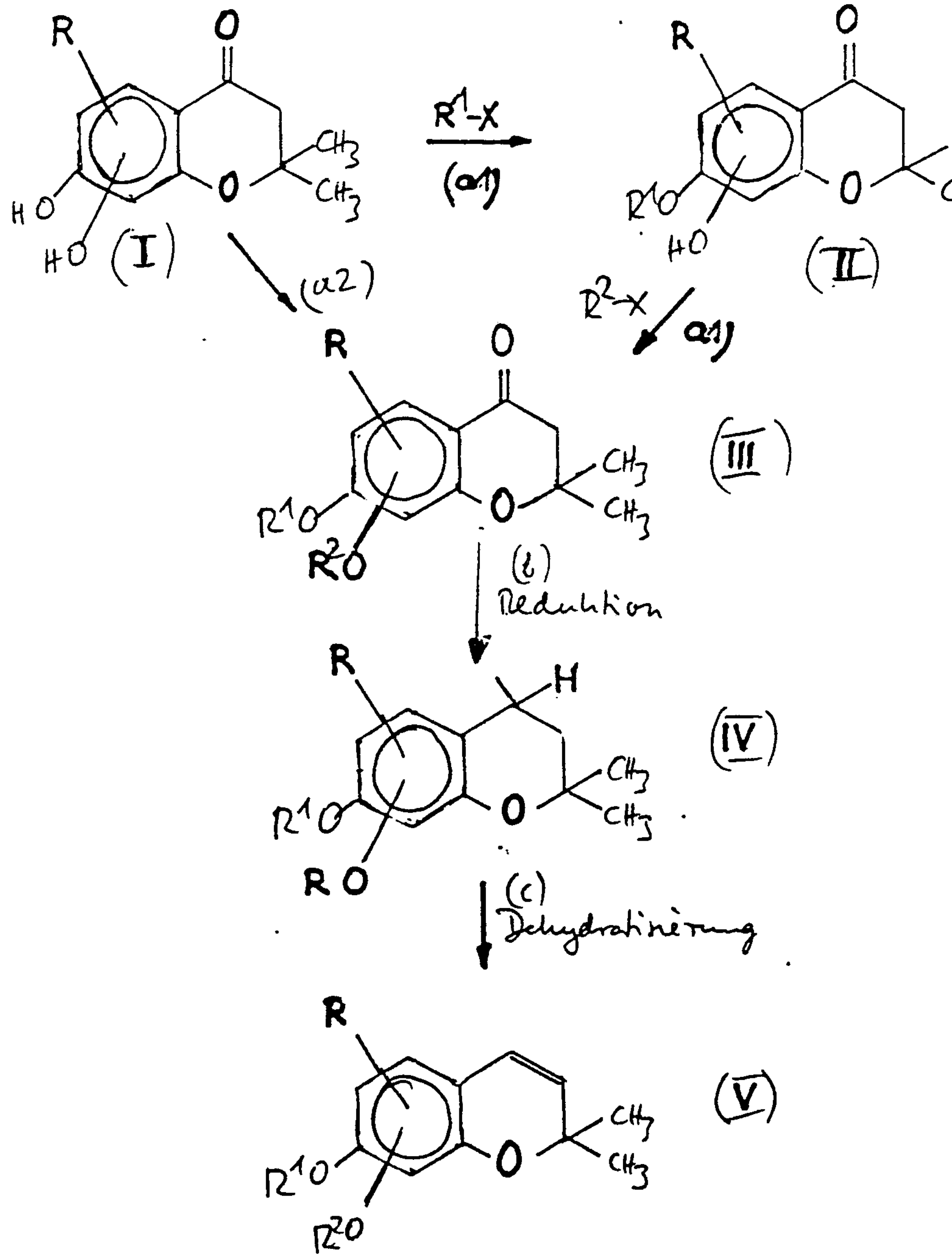

Die Erfindung betrifft ferner die Verbindungen der Formel I als Zwischenprodukte zur Synthese entsprechender Chromenderivate der Formel V.

Die Erfindung beruht auf der Erkenntnis, daß die Hydroxylgruppen der Hydroyx-4-chromanone der allgemeinen Formel I infolge ihrer Wechselwirkung mit der 4-Carbonylgruppe bei O-Substituionsredaktionen eine unterschiedliche Reaktivität aufweisen. Dieser Unterschied kann durch gezielte Wahl der Reaktionsbedingungem verstärkt werden, wodurch die selektive Mono-O-substitution in günstiger Ausbeute verläuft. Durch unterschiedliche Messungen und Berechnungen ($^1$H-NMR, $^{13}$C-NMR, UV-VIS-Spektrophotometrie, potentiometreische Säure-Basen-Titration, pK-Bestimmung, CNDO, quantenchemische "All-valence"-Berechnung des elektrostatischen Potentialbildes) wurde nachgewiesen, daß die Hydroxylgruppe in 7-Stellung am reaktivsten ist, während die Reaktivität der Hydroxylgruppen in 5-, 6- und 8-Stellung geringer ist. Dieser Unterschied wird durch die in der Tabelle 1 zusammengefaßten, im Rahmen der vorliegenden Erfindung bestimmten beziehungsweise berechneten pK-Werte veranschaulicht.

**Tabelle 1**

| Substituenten | $pK_7$ (1) | $pK_7$ (2) | $pK_x$ (2) |
|---|---|---|---|
| 5-OH 7-OH 4-H | 7,4 | 7,0 | 11,6 (X = 5) |
| 6-OH 7-OH 4-H | 6,8 | 6,9 | 11,4 (X = 6) |
| 8-OH 7-Oh 4-H | 7,2 | 7,2 | 11,6 (X = 8) |

(1): potentiometrisch titriert
(2): durch spektrophotometrische Messung

Daher kann die 7-Hydroxygruppe des 6,7-Dihydroxy-2,2-dimethyl-4-chromanons mit 90- bis 95-%-iger präparativer Ausbeute selektiv alkyliert werden. Dies eröffnet die Möglichkeit, aus dem erhaltenen Derivat die 6-Hydroxy-7-alkoxy-chromanone und dann mit anderen Alkylierungsmitteln oder Acylierungsmitteln gemischte 6,7-Dialkoxy- oder 6-Acyloxy-7-alkoxy-chromanone zu erhalten.

Die Erfindung ermöglicht es, wirksamere Analoga als die bekannten Precocene aus leicht zugänglichen Ausgangsstoffen einheitlichen Typs mit bekannten und sehr billigen Reagentien herzustellen. Das Verfahren ist ferner industriell einfach, billig und universell anwendbar und geeignet, die Synthese dieser Verbindungen und ihre Anwendung in der Schädlingsbekämpfung zu revolutionieren.

Die Erfindung wird im folgenden anhand von Ausführungsbeispeilen näher erläutert. Die Reinheit der in den Beispielen genannten Verbindungen wurde dünnschichtchromatographisch und gaschromatographisch kontrolliert. Die angegebenen Schmelzpunkte sind nicht korrigiert. Die Struktur der Verbindungen wurde durch UV-, IR-, $^1$H-NMR- und $^{13}$C-NMR-Spektren und fallweise auch durch Elementaranalyse bestätigt.

Bei der Angabe der H-NMR-Spektren sind folgende Symbole verwendet:

Beispiel: 1,45 (3H, t, J = 5 Hz):
- 1,45 chemische Verschiebung,
- 3H Anzahl der zu dem Signal gehörenden Protonen,
- t Multiplizität des Signals,
- J Kupplungskonstante.

Kennzeichnung der Multipletts: s = Singulett, d = Dublett, t = Triplett, q = Quartett, m = Multiplett höherer Ordnung, b = breites Signal.

**1. Herstellung von Verbindungen der Formel II:**

**Beispiel 1**

6-Hydroxy-7-methoxy-2,2-dimethyl-4-chromanon

In 100 ml Aceton werden 4,2 g (20 mmol) 6,7-Dihydroxy-2,2-dimethyl-4-chromanon gelöst; zu der Lösung werden unter Rühren 4,1 g (30 mmol) Kaliumcarbonat und 4,5 g (2 ml; 30 mmol) Methyljodid gegeben. Das Gemisch wird 4 h am Rückfluß gekocht. Dann wird das anorganische Salz abfiltriert und mit 2 x 20 ml Aceton gewaschen. Das mit der Waschflüssigkeit vereinigte Filtrat wird vom Lösungsmittel befreit. Der Rückstand wird in 100 ml Chloroform gelöst und die Lösung mit 2 x 50 ml 4-%-iger Natronlauge ausgeschüttelt. Die wäßrig-alkalische Phase wird abgetrennt, auf 5°C gekühlt und unter ständigem Rühren mit 37-%-iger Salzsäure angesäuert. Das sich ausscheidende Produkt wird abfiltriert, mit 2 x 20 ml Wasser gewaschen und bis zur Massenkonstanz getrocknet. Das Produkt wird durch Umkristallisieren aus abs. Ethanol gereinigt.

Ausbeute: 4,0 g (90 %).
F: 134 - 136° C.
IR ($CCl_4$): CO: 1690 $cm^{-1}$. OH: 3570 $cm^{-1}$.
NMR ($CDCl_3$): 1,4 (6H, s), 2,62 (2H, s), 3,88 (3H, s), 6,42 (2H, b.+s), 7,36 (1H, s).

**Beispiel 2**

7-Ethoxy-6-hydroxy-2,2-dimethyl-4-chromanom

In 100 ml Methylethylketon werden 4,2 g (20 mmol) 6,7-Dihydroxy-2,2-dimethyl-4-chromanom gelöst; zu der Lösung werden 4,1 g (30 mmol) Kaliumcarbonat und 4,7 g (2,4 ml; 30 mmol) Ethyljodid gegeben. Das Gemisch wird 3 Stunden lang gekocht und dann auf die im Beispiel 1 angegebene Weise aufgearbeitet.

Ausbeute: 4,0 g (85 %).
F: 129 - 130° C.
IR ($CCl_4$): CO: 1690 $cm^{-1}$, OH: 3570 $cm^{-1}$.
NMR ($CDCl_3$): 1,5 (9H, m), 2,64 (2H, s), 4,18 (2H, q, J = 8 Hz), 6,41 (2H, b.+s), 7,4 (1H, s).

**Beispiel 3**

6-Hydroxy-7-i-propoxy-2,2-dimethyl-4-chromanon

In 80 ml N,N-Dimethylformamid werden 4,2 g (20 mmol) 6,7-Dihydroxy-2,2-dimethyl-4-chromanon gelöst; zu der Lösung werden 4,1 g (30 mmol) Kaliumcarbonat und 4,2 g (2,5 ml; 25 mmol) i-Propyljodid gegeben. Das Gemisch wird 6 h auf 130° C gehalten, dann mit 200 ml Wasser verdünnt und mit 100 ml Tetrachlorkohlenstoff ausgeschüttelt. Die wäßrige Phase wird mit 50 ml 5-%-iger Natronlauge versetzt und auf 5° C abgekühlt. Nach Ansäuren mit konzentrierter Salzsäure fällt das Produkt kristallin aus. Es wird aus Mehtanol umkristallisiert.

Ausbeute: 4,5 g (90 %).
F: 138 - 139° C,
IR ($CCl_4$). CO: 1690 $cm^{-1}$, OH: 3565 $cm^{-1}$
NMR ($CDCl_3$): 1,48 (12H, m), 2,66 (2H, s), 4,83 (1H, m), 6,0 (1H, b.), 6,54 (1H, s), 7,52 (1H, s).

**Beispiel 4**

7-s-Butoxy-6-hydroxy-2,2-dimethyl-4-chromanon

4,2 g (20 mmol) 6,7-Dihydroxy-2,2-dimethyl-4-chromanon werden in 100 ml Methylethylketon gelöst; zu der Lösung werden unter Rühren 4,1 g (30 mmol) Kaliumcarbonat, 0,5 g Kaliumjodid und 3,3 g (2,6 ml; 24 mmol) s-Butylbrodmid gegeben. Das Gemisch wird 12 h gekocht und denn auf die in Beispiel 1 beschriebene Weise aufgearbeitet.

Ausbeute: 4,5 g (86 %).
F: 114 - 116° C.
IR ($CCl_4$): CO: 1690 $cm^{-1}$ OH: 3560 $cm^{-1}$.
NMR ($CDCl_3$): 0,97 (3H, t, J = 6 Hz), 1,32 (3H, d, J = 6 Hz), 1,43 (6H, s), 1,8 (2H, m), 2,63 (2H, s), 4,4 (1H, m), 6,13 (1H, b.), 6,4 (1H, s), 7,36 (1H, s).

**Beispiel 5**

7-i-Butoxy-5-Hydroxy-2,2-dimethyl-4-chromanon

In 50 ml Diethylenglyckoldimethylehter werden 4,2 g (20 mmol) 5,7-Dihydroxy-2,2-dimethyl-4-chromanon gelöst; zu der Lösung werden 3,0 g (22 mmol) Kaliumcarbonat, 0,2 g Kaliumjodid und 3,3 g (2,6 ml; 25 mmol) i-Butylbromid gegeben. Das Gemisch wird 10 h auf 100°C gehalten und dann auf die in Beispiel 3 beschriebene Weise aufgearbeitet.

Ausbeute: 4,17 g (79 %).
F.: 77 - 79°C.
NMR ($CDCl_3$): 0,96 (6H, d, J = 6 Hz), 1,4 (6H, s), 2,0 (1H, m), 2,6 (2H, s), 3,66 (2H, d, J = 6 Hz), 5,9 (2H, m), 12,2 (1H, b.).

**Beispiel 6**

8-Hydroxy-7-i-propoxy-2,2-dimethyl-4-chromanon

4,2 g (20 mmol) 7,8-Dihydroxy-2,2-dimethyl-4-chromanon werden in 50 ml N,N-Dimethylformamid gelöst; zu der Lösung werden 5 ml Diethylenglycoldimethyleter und anschließend 3,0 g (22 mmol) Kaliumcarbonat sowie 4,2 g (2,5 ml; 25 mmol), i-Propyljodid gegeben. Das Gemisch wird 4 h auf 140°C gehalten und dann auf die in Beispiel 3 beschriebene Weise aufgearbeitet.

Ausbeute: 4,5 g (90 %).
F.: 114 - 116°C.
NMR ($CDCl_3$): 1,36 (6H, d, J = 6 Hz), 1,46 (6H, s), 2,66 (2H, s), 4,6 (1H, m), 6,0 (1H, b.), 6,5 (1H, d, J = 8 Hz), 7,34 (1H, d, J = 8 Hz).

**Beispiel 7**

7-s-Butyloxy-8-hydroxy-2,2-dimethyl-4-chromanon

4,2 g (20 mmol) 7,8-Dihydroxy-2,2-dimethyl-4-chromanon werden in 80 ml Dimethylsulfoxyd gelöst; zu der Lösung werden 10 ml Diethylenglycoldimethyleter, 4,1 g (30 mmol) Kaliumcarbonat, 0,5 g Kaliumjodid und 3,3 g (2,6 ml; 24 mmol) s-Butylbromid gegeben. Das Gemisch wird 10 h auf 100°C gehalten und dann auf 200 ml Eisstücke gegossen. Nach Extrahieren mit 100 ml Tetrachlorkohlenstoff und Trennen der Phasen wird die wäßrige Phase mit 20 ml 10-%-iger Natronlauge versetzt, auf 0°C gekühlt, dann mit konzentrierter Salzsäurre angesäuert und bei 0°C 2 h gerührt. Das ausgefallene Produkt wird abfiltriert, mit 2 x 20 ml Wasser gewaschen, getrocknet und aus 90-%-igem Ethanol umkristallisiert.

Ausbeute: 4,4 g (83 %).
F.: 108 - 110°C.
NMR ($CDCl_3$): 0,96 (3H, t, J = 8 Hz), 1,3 (3H, d, J = 5 Hz), 1,44 (6H, s), 1,7 (2H, m), 2,66 (2H, s), 4,3 (1H, m), 5,9 (1H, b.), 6,5 (1H, d, J = 8 Hz), 7,3 (1H, d, J = 8 Hz).

**2. Herstellung von Chromanonen der allgemeinen Formel III:**

**Beispiel 8**

6,7-(1,3-Propylendioxy)-2,2-dimethyl-4-chromanon

Zu einer auf 120°C erwärmten Lösung von 6 g (3 ml; 30 mmol) 1,3-Dibrompropan in 50 ml N,N-Dimethylformamid wird unter Rühren innerhalb von 3 Stunden eine Suspension von 4,2 g 6,7-Dihydroxy-2,2-dimethyl-4-chromanon, 8,3 g (60 mmol) Kaliumcarbonat und 0,5 g Kaliumjodid in 50 ml N,N-Dimethylformamid tropfenweise zugegeben. Das Reaktionsgemisch wird weitere 2 h auf 120°C gehalten und dann mit 100 ml Wasser verdünnt und mit 2 x 50 ml Chroloform extrahiert. Die organische Phase wird mit 2 x 50 ml Wasser

gewaschen, über Natriumsulfat getrocknet, worauf das Lösungsmittel entfernt und der Rückstand aus Hexan kristallisiert wird.

Ausbeute: 3,7 g (75 %) eines gelben Öls.
NMR ($CDCl_3$): 1,36 (6H, s), 2,1 (2H, m), 2,58 (2H, s), 4,2 (4H, m), 6,4 (1H, s), 7,36 (1H, s).

**Beispiel 9**

6-Ethoxy-7-methoxy-2,2-dimethyl-4-chromanon
4,45 g (20 mmol) 6-Hydroxy-7-methoxy-2,2-dimethyl-4-chromanon werden in 100 ml Methylethylketon gelöst und zu einer Lösung 4,1 g (30 mmol) Kaliumcarbonat und 4,7 g (2,4 ml; 30 mmol) Ethyljodid gegeben. Das Gemisch wird 5 h am Rückfluß gekockt; dann wird das anorganische Salz abfiltriert, mit 2 x 20 ml Aceton gewaschen und das mit der Waschflüssigkeit vereinigte Filtrat vom Lösungsmittel befreit. Der Rückstand wird aus 90-%-igem Ethanol umkristallisiert.

Ausbeute: 4,75 g (95 %).
F.: 86 - 87°C.
NMR ($CDCl_3$): 1,44 (9H, s+t, J = 6 Hz), 2,68 (2H, s), 3,92 (3H, s), 4,14 (2H, q, J = 6 Hz), 6,44 (1H, s), 7,32 (1H, s).

**Beispiel 10**

7-Ethoxy-6-methoxy-2,2-dimethyl-4-chromanon
4,7 g (20 mmol) 7-Ethoxy-6-hydroxy-2,2-dimethyl-4-chromanon werden in 100 ml Aceton gelöst; zu der Lösung werden 4,1 g (30 mmol) Kaliumcarbonat und 4,5 g (2 ml; 30 mmol) Methyljodid gegeben. Das Gemisch wird 4 h am Rückfluß gekockt. Dann wird das anorganische Salz abfiltriert, mit 2 x 20 ml Aceton gewaschen und das mit der Waschflüssigkeit vereinigte Filtrat auf die Hälfte seines Volumens eingedampft. Der verbleibende Teil wird auf 0°C abgekühlt, wobei sich das Produkt abscheidet. Es wird abfiltriert und bis zur Massenkonstanz getrocknet.

Ausbeute: 4,6 g (92 %).
F.: 120 - 122°C.
NMR ($CDCl_3$): 1,5 (9H, m), 2,64 (2H, s), 3,86 (3H, s), 4,16 (2H, q, J = 8 Hz), 6,4 (1H, s), 7,32 (1H, 2).

**Beispiel 11**

7-s-Butoxy-6-methoxy-2,2-dimethyl-4-chromanon
5,2 g (20 mmol) 7-s-Butoxy-6-methoxy-2,2-dimethyl-4-chromanon werden in 40 ml 5-%-iger Natronlauge gelöst; zu der Lösung werden 80 ml Dichlormethan und 0,5 g Triethylbenzylaminiumchlorid gegeben. Das Gemisch wird bei Raumtemperatur 30 Minuten lang intensiv gerührt, dann mit 4,25 g (1,9 ml; 30 mmol) Methyljodid versetzt und weitere 2 h gerührt. Die organische Phase wird abgetrennt, mit 2 x 50 ml Wasser gewaschen, über Natriumsulfat getrocknet und dann eingedampft. Der Rückstand wird aus 90-%-igem Ethanol umkristallisiert.

Ausbeute: 5,34 g (96 %).
F.: 92,5 - 93°C.
NMR ($CDCl_3$): 0,96 (3H, t, J = 6 Hz), 1,34 (3H, d, J = 4 Hz), 1,4 (6H, s), 1,7 (2H, m), 2,6 (2H, s), 3,8 (3H, s), 4,3 (1H, m), 6,36 (1H, s), 7,22 (1H, s).

**Beispiel 12**

7-s-Butoxy-5-methoxy-2,2-dimethyl-4-chromanon

5,6 g (20 mmol) 7-s-Butoxy-5-hydroxy-2,2-dimethyl-4-chromanon-Na werden in 100 ml Acetonitril gelöst und in Gegenwart von 0,5 g (2 mmol) 18-Krone-6 30 min gerührt. Nach Zusatz von 4,25 g (1,9 ml; 30 mmol) Methyljodid wird noch 3 h gerührt; dann wird das anorganische Salz abfiltriert und das Filtrat vom Lösungsmittel befreit. Der Rückstand wird in Tetrachlorkohlenstoff gelöst, die Lösung mit 2 x 30 ml Wasser gewaschen, über Natriumsulfat getrocknet und schließlich eingedampft.

Ausbeute: 5,1 g (92 %), hellgelbes Öl.

NMR ($CDCl_3$): 0,96 (3H, t, J = 7 Hz), 1,3 (3H, d, J = 8 Hz), 1,44 (6H, s), 1,7 (2H, m), 2,6 (2H, s), 3,85 (3H, s), 4,3 (1H, m), 6,0 (2H, m).

**Beispiel 13**

7-i-Butoxy-8-methoxy-2,2-dimethyl-4-chromanon

5,2 g (20 mmol) 7-i-Butoxy-8-hydroxy-2,2-dimethyl-4-chromanon werden unter Stickstoffatmosphäre in 50 ml 10-%-iger Natronlauge gelöst; zu der Lösung werden 2,6 g (2 ml, 21 mmol) Dimethylsulfat gegeben. Das Gemisch wird bei 50°C 3 Stunden lang intensiv gerührt, dann mit 100 ml Eiswasser verdünnt und mit 3 x 50 ml Chloroform extrahiert. Die organische Phase wird mit 2 x 40 ml Wasser gewaschen, über Natriumsulfat getrocknet und dann vom Lösungsmittel befreit.

Ausbeute: 5,2 g (93 %), gelbes Öl.

NMR ($CDCl_3$): 0,96 (6H, d, J = 8 Hz), 1,4 (6H, s), 2,1 (1H, m), 2,6 (2H, s), 3,8 (3H, s), 3,88 (2H, m), 6,5 (1H, d, J = 8 Hz), 7,56 (1H, d, J = 8 Hz).

**3. Herstellung von 2H-Chromenen der allgemeinen Formel V:**

**Beispiel 14**

7-Ethoxy-6-methoxy-2,2-dimethyl-2H-chromen (P3)

5,0 g (20 mmol) 7-Ethoxy-6-methoxy-2,2-dimethyl-4-chromanon wrden in 80 ml wasserfreiem Tetrahydrofuran gelöst; zu der Lösung werden in winzigen Portionen unter Rühren 1,5 g Lithiumalanat zugegeben. Das Gemisch wird 1 h gekocht. Dann wird das Gemisch auf 15°C abgekühlt, tropfenweise mit 50 ml 4 n Salzsäure versetzt und bei maximal 20°C 30 min gerührt. Das zweiphasige System wird in die Phasen getrennt; die wäßrige Phase wird mit 2 x 40 ml Petrolether extrahiert; die vereinigten organischen Phasen werden mit 2 x 25 ml 5-%-iger Natronlauge und danach mit 2 x 40 ml Wasser gewaschen, über Natriumsulfat getrocknet und dann eingedampft. Der Rückstand wird aus 80-%-igem Methanol umkristallisiert.

Ausbeute: 4,1 g (88 %), farbloses Öl.

NMR ($CDCl_3$): 1,45 (9H, m), 3,8 (3H, s), 4,1 (2H, q, J = 8 Hz), 5,48 (1H, d, J = 10 Hz), 6,28 (1H, d, J = 10 Hz), 6,46 (1H, s), 6,6 (1H, s).

**Beispiel 15**

7-s-Butoxy-5-methoxy-2,2-dimethyl-2H-chromen

In einem Gemisch aus 60 ml Tetrahydrofuran und 40 ml Methanol werden 5,6 g (20 mmol) 7-s-Butoxy-5-methoxy-2,2-dimethyl-4-chromanon gelöst; zu der Lösung werden in kleinen Portionen 1,5 g (40 mmol) Natriumtetrahydroboranat gegeben. Das Gemisch wird 2 h gekockt und dann auf die im Beispiel 14 beschriebene Weise aufgearbeitet. Das Produkt wird säulenchromatographisch gereinigt.

Ausbeute: 4,4 g (85 %), hellgelbes Öl.

NMR ($CDCl_3$): 0,92 (3H, t, J = 8 Hz), 1,24 (3H, d, J = 8 Hz), 1,36 (6H, s), 1,6 (2H, m), 3,7 (3H, s), 4,2 (1H, m), 5,3 (1H, d, J = 10 Hz), 5,92 (2H, m), 6,5 (1H, d, J = 10 Hz).

**Beispiel 16**

7-s-Butoxy-8-methoxy-2,2-dimethyl-2H-chromen

5,6 g (20 mmol) 7-s-Butoxy-8-methoxy-2,2-dimethyl-4-chromanon werden in 100 ml Tetrahydrofuran gelöst; zu der Lösung werden unter Rühren 1,5 g Lithiumalanat in kleinen Portionen zugegeben. Das Gemisch wird 2 h gekocht und dann auf die im Beispiel 14 beschriebene Weise aufgearbeitet. Das Produkt wird säulenchromatographisch gereinigt.

Ausbeute: 4,2 g (80 %), farbloses Öl.

NMR ($CDCl_3$): 0,96 (3H, t, J = 8 Hz), 1,24 (3H, d, J = 8 Hz), 1,4 (6H, s), 1,7 (2H, m), 3,76 (3H, s), 4,2 (1H, m) 5,4 (1H, d, J = 10 Hz), 6,16 (1H, d, J = 10 Hz), 6,32 (1H, d, J = 8 Hz), 6,56 (1H, d, J = 8 Hz).

Zur Säulenchromatographie wurden als Adsorbens Kieselgel 60 und als Elutionsmittel Hexan und Ether im Verhältnis 9 : 1 verwendet.

**Beispiel 17**

Auf die beschriebene Weise wurden folgende Verbindungen der Formel V hergestellt:

7-s-Butoxy-6-methoxy-2,2-dimethyl-2H-chromen
7-i-Butoxy-6-methoxy-2,2-dimethyl-2H-chromen
7-Benzyloxy-6-methoxy-2,2-dimethyl-2H-chromen
7-Ethoxy-5-methoxy-2,2-dimethyl-2H-chromen
5-Methoxy-7-n-propoxy-2,2-dimethyl-2H-chromen
5-Methoxy-7-i-propoxy-2,2-dimethyl-2H-chromen
7-s-Butoxy-5-methoxy-2,2-dimethyl-2H-chromen
7-i-Butoxy-5-methoxy-2,2-dimethyl-2H-chromen
7-Ethoxy-8-methoxy-2,2-dimethyl-2H-chromen
8-Methoxy-7-n-propoxy-2,2-dimethyl-2H-chromen
8-Methoxy-7-i-propoxy-2,2-dimethyl-2H-chromen
7-s-Butoxy-8-methoxy-2,2-dimethyl-2H-chromen
7-i-Butoxy-8-methoxy-2,2-dimethyl-2H-chromen
6-Ethoxy-7-methoxy-2,2-dimethyl-2H-chromen
6-Methoxy-7-i-propoxy-2,2-dimethyl-2H-chromen
7-Methoxy-6-i-propoxy-2,2-dimethyl-2H-chromen
6-Methoxy-7-n-propoxy-2,2-dimethyl-2H-chromen
7-n-Butoxy-6-methoxy-2,2-dimethyl-2H-chromen
7-n-Hexyloxy-6-methoxy-2,2-dimethyl-2H-chromen

**Beispiel 18**

Formulierung der Precocen-Analoga der Formel V als 50 EC.

Auf 500 g 100-%-igen Wirkstoff der allgemeinen Formel V werden 64,2 g "Arylan" C.A. und 40,0 g "Lubrol" N 15 umgesetzt, worauf mit dem Lösungsmittel Aromasol auf 1000 ml aufgefüllt wird. Aus dieser Zubereitung werden durch Verdünnen mit Wasser auf die gewünschte Konzentration die zum Testen der Verbindungen der allgemeinen Formel V verwendeten Anwendungsformen hergestellt.

**Beispiel 19**

Untersuchung der auf die Insekten ausgeübten entwicklungshemmenden Wirkung und der nematociden Wirkung von Precocen-Analoga der allgemeinen Formel V.

Testtiere:

| | |
|---|---|
| Insekten: | Baumwollwanze (Dysdercus fasciatus) |
| | Kohlweißling (Pieris brassicae) |
| | Senfkäfer |
| | Stubenfliege (Musca domestica) |
| | Erbsenblattlaus (Acirtosyphon pisi) |
| Fadenwürmer: | Caenorhabditis elegans. |

**Durchführung der Versuche**

1. Baumwollwanze

Mit den zu untersuchenden Verbindungen wurden mit Aceton Verdünnungsreihen hergestellt. Von jeder Verdünnung wurden mit einer Spritze jeweils 0,2 µl auf die Rückseite von im Stadium II befindlichen Larven aufgebracht. Der Wirkstoff wurde durch die Cuticula hindurch absorbiert. Die Insekten wurden in großen Mäusegläsern gehalten und mit Baumwollkernen und Wasser ernährt. Nach der adulten Häutung wurden die überlebenden Tiere auf eventuell vorhandene adultoide Merkmale untersucht; ihre Eierproduktion und das Ausschlüpfen aus den Eiern wurden beobachtet.

2. Kohlweißling

Aus 50 EC-Formulierungen der zu untersuchenden Verbindungen wurden mit Wasser Verdünnungsreihen bereitet; mit jeder Verdünnung wurden junge Kohlplanzen tropfnaß eingesprüht. Die Pflanzen wurden nach dem Trocknen mit je 20 im Stadium II befindlichen Larven besetzt. Die Pflanzen wurden bei täglich 18 Stunden Licht und 6 Stunden Dunkelheit gehalten. Die abgenagten Fraßpflanzen wurden nach Notwendigkeit gegen ebenso durch Sprühen vorbehandelte Pflanzen ausgewechselt. Beobachtet wurden die Mortalität, die Geschwindigkeit der Larvenentwicklung sowie eventuelle morphologische Mißbildungen der überlebenden Puppen und Adulte.

3. Senfkäfer

Aus 0,1 g Testverbindung, 0,5 ml Dimethylsofoxid und 10 µl 50 EC-Formulierung sowie 10 ml Wasser wurde eine Testformulierung hergestellt und auf zwei Wochen alte Senfpflanzen gesprüht. Nachdem die Pflanzen abgetrocknet waren, wurde jede mit 20 im Larvenstadium II befindlichen Larven des Senfkäfers besetzt. Die Fraßpflanzen wurden alle zwei Tage gewechselt. Zur Auswertung wurde die Anzahl der aus den Puppen geschlüpften Imagines bestimmt.

4. Stubenfliege

Zur Anzucht der Fliegenlarven verwendeter Nährboden: 2 ml Milch, 2 ml Wasser, 2 g Weizenkleie, 0,1 ml gesättigte alkoholische Nipaginlösung. Der Nährboden wurden in zylindrischen Glasgefäßfen (30 x 100 mm) gemischt; in jedem Gefäß wurden 25 Fliegenlarven gezogen. Die Wirkstoffe wurden in der Milch zu einer 0,1-%-igen Lösung gelöst, was auf den gesamten Nährboden bezogen eine Konzentration von 0,05 % ergab. Die 25 Larven (I. Stadium, frisch geschlüpft) wurden nach dem Zumischern des Wirkstoffes in das Gefäß gegeben, das dann mit einem harten Wattestopfen verschlossen wurde. Nachdem sich die Larven verpuppt hatten, wurden sie in Petrischalen (Durchmesser 25 mm) gelegt und dort bis zum Schlüpfen belassen. Die Anzahl der aus den Puppen schlüpfenden Fliegen wurde bestimmt.

5. Caenorhabditis elegans

Auf eine bakterienfreie Agarplatte, die sich in einer Petrischale befand, wurden 0,5 ml einer acetonischen Lösung der zu untersuchenden Verbindung aufgebracht. nachdem das Aceton getrocknet war, wurden 25 - 30 junge Adulte auf die Platte gesetzt. Nach 24 Stunden wurden die lebenden und die toten Tiere gezählt.

**Tabelle 4** Zu den biologischen Versuchen verwendete Verbindungen der Formel V

| Verbindung Nr. | |
|---|---|
| 1 | 6-Methoxy-7-i-propoxy-2,2-dimethyl-2H-chromen |
| 2 | 7-s-Butoxy-6-methoxy-2,2-dimethyl-2H-chromen |
| 3 | 7-i-Butoxy-6-methoxy-2,2-dimethyl-2H-chromen |
| 4 | 6-Ethoxy-7-methoxy-2,2-dimethyl-2H-chromen |
| 5 | 5-Methoxy-7-n-propoxy-2,2-dimethyl-2H-chromen |
| 6 | 5-Methoxy-7-i-propoxy-2,2-dimethyl-2H-chromen |
| 7 | 7-s-Butoxy-5-methoxy-2,2-dimethyl-2H-chromen |
| 8 | 7-i-Butoxy-5-methoxy-2,2-dimethyl-2H-chromen |
| 9 | 7-Ethoxy-8-methoxy-2,2-dimethyl-2H-chromen |
| 10 | 8-Methoxy-7-n-propoxy-2,2-dimethyl-2H-chromen |

| | |
|---|---|
| 11 | 8-Methoxy-7-i-propoxy-2,2-dimethyl-2H-chromen |
| 12 | 7-s-Butoxy-8-methoxy-2,2-dimethyl-2H-chromen |
| 13 | 7-i-Butoxy-8-methoxy-2,2-dimethyl-2H-chromen |

Die Ergebnisse der Tests sind in den folgenden Tabellen 5 - 9 angegeben. In den Tests mit der Baumwollwanze wurden jeweils 50 Insekten verwendet. Die Tabellen enthalten auch die Ergebnisse von Kontrollversuchen.

**Tabelle 5**

Wirkung auf Baumwollwanzen

| Verbindung | $LD_{50}$ | Antijuvenilhormonwirkung (µg/Tier) | Sterilisation |
|---|---|---|---|
| P2 | 0,6 | 1 | 10 |
| 1 | 0,45 | 1 | 0,2 |
| 2 | 0,4 | 0,1 | - |
| 3 | 0,4 | - | - |
| Kontrolle+) | - | - | - |

+) Behandlung mit Aceton, 90- bis 95-%-iges Überleben.

**Tabelle 6**

Wirkung auf Kohlweißlinge

| Verbindung | Absterben der Larven (%) | Überleben Puppe (%) | Überleben Adulte (%) |
|---|---|---|---|
| P1 | 41 | 59 | 50 |
| 5 | 88 | 12 | 12 |
| 6 | 87 | 13 | 9 |
| 7 | 100 | - | - |
| 8 | 100 | - | - |
| 9 | 76 | 24 | 24 |
| 10 | 100 | - | - |
| 11 | 64 | 36 | 32 |
| 12 | 100 | - | - |
| 13 | 93 | 7 | 7 |
| Kontrolle+) | 12 | 88 | 88 |

+) 50 EC-Formulierung ohne Wirkstoff.

Die Wirkstoffe wurden in einer Konzentration von 0,1 % formuliert.

**Tabelle 7**

Wirkung auf Senfkäfer

| Verbindung | Konzentration (%) | Überlebensrate Adulte (%) |
|---|---|---|
| 10 | 1 | 45 |
| Kontrolle | 0 | 100 |

**Tabelle 8**

Wirkung der Precocen-Analoga auf die Stubenfliege

| Verbindung | Überleben Puppe (%) | Überleben Adulte (%) |
|---|---|---|
| P1 | 100 | 33,3 |
| 8 | 85 | 12,8 |
| 5 | 9,1 | 6,4 |
| 8 | 0 | 0 |
| 9 | 5,4 | 4,2 |
| 10 | 12,7 | 0 |
| 11 | 12 | 0 |
| Kontrolle | 100 | 100 |

**Tabelle 9**

Wirkung der Precocen-Analoga auf Caenorhabditis elegans (Adult)

| Verbindung | Konzentr. ωg/ml | Letalität % | Nachkommenschaft | Bemerkung |
|---|---|---|---|---|
| P1 | 200 | 44 | reduziert | |
| | 400 | 98 | keine | |
| P2 | 200 | 100 | keine | |
| | 400 | 100 | keine | |
| P3 | 200 | 70 | wenige Nachkommen | |
| | 400 | 90 | keine | |
| 1 | 200 | 70 | reduziert | |
| | 400 | 80 | keine | |
| 4 | 200 | 15 | reduziert | Lähmung |
| | 400 | 23 | wenig N. | Lähmung |
| 9 | 200 | 40 | reduziert | |
| | 400 | 100 | keine | |
| 11 | 200 | 60 | reduziert | |
| | 400 | 73 | reduziert | |
| 12 | 200 | 25 | reduziert | |
| | 400 | 85 | reduziert | |
| Kontrolle | | 0 | normal | |
| 10 % Aceton | | 0 | normal | |

**Patentansprüche**

1. Verfahren zur Herstellung von Chromenderivaten der allgemeinen Formel V,

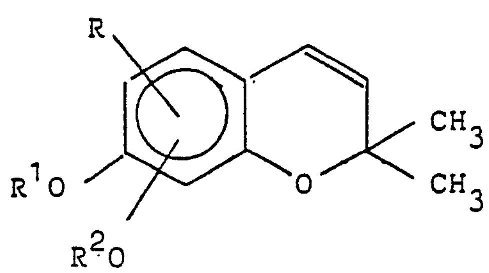

(V),

worin bedeuten:
R Wasserstoff oder $C_{1-8}$-Alkyl und
$R^1$ und $R^2$ $C_{1-10}$-Alkyl, Benzyl, Trifluorethyl oder Acetyl, wobei $R^1$ und $R^2$ verschieden sind, oder zusammen eine Gruppe $-(CH_2)_3-$,
<u>gekennzeichnet durch</u>

(a1) Umsetzung eines Chromanonderivats der allgemeinen Formel I

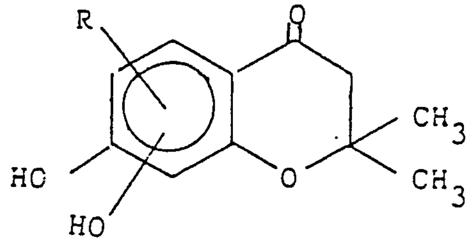

(I)

mit R wie oben
mit einem Reagens der allgemeinen Formel

$R^1$-X,
>R worin
$R^1$ dasselbe wie oben und
X den Rest eines Alkylierungsmittels oder Acetylierungsmittels bedeuten,
in einer etwa äquivalenten Menge zu einem mono-O-substituierten Chromanonderivat der allgemeinen Formel II

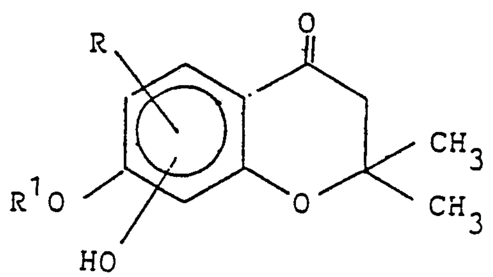

(II)

mit R und $R^1$ wie oben
und
Umsetzung des Chromanonderivats der Formel II mit einem Reagens der allgemeinen Formel

$R^2$-X

mit $R^2$ und X wie oben in einer Menge von 1,1 bis 1,5 mol pro Mol des Chromanonderivats der Formel II zu einem di-O-substituierten Chromanonderivat der allgemeinen Formel III

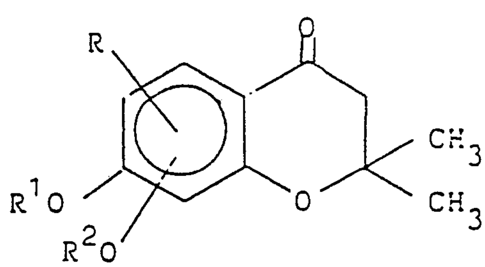

(III)

mit R, $R^1$ und $R^2$ wie oben
oder alternativ
(a2) direkte Umsetzung des Chromanonderivats der Formel I mit 1,3-Dibrompropan zum Chromanonderivat der Formel III, in der $R^1$ und $R^2$ zusammen $-(CH_2)_3-$ bedeuten,
(b) an sich bekannte Reduktion des Chromanonderivats der Formel III zum entsprechenden Chromanol der allgemeinen Formel IV

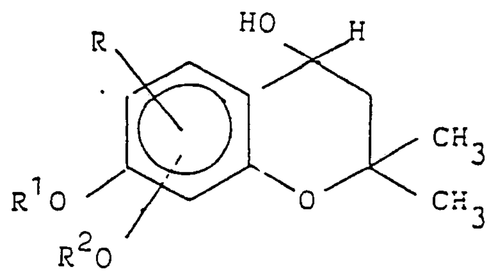

(IV)

mit R, $R^1$ und $R^2$ wie oben und

(c) an sich bekannte Dehydratisierung des Chromanols der Formel IV in einem wäßrigen, sauren Medium.

2. Verfahren nach Anspruch 1, dadurch gekennzeichnet, daß bei Variante (a1) als Alkylierungsmittel entsprechende Alkylhalogenide, Dialkylsulfate oder Trifluorethyltosylat eingesetzt werden.

3. Verfahren nach Anspruch 1 oder 2, dadurch gekennzeichnet, daß in Variante (a1) als Alkylierungsmittel Methyljodid, Ethyljodid oder Isopropyljodid oder Dimethylsulfat eingesetzt werden.

4. Verfahren nach einem der Ansprüche 1 bis 3, dadurch gekennzeichnet, daß zur Herstellung der Chromanone der allgemeinen Formel II nach Variante (a1) das Reagens der Formel

$R^1$-X

mit $R^1$ und X wie oben in einer Menge von 0,8 bis 1,5 mol pro Mol der Verbindung der Formel I eingesetzt wird.

5. Verfahren nach einem der Ansprüche 1 bis 4, dadurch gekennzeichnet, daß die mono-O-substituierten Chromanonderivate der allgemeinem Formel II in Form ihrer Salze gereinigt werden.

6. Verfahren nach einem der Ansprüche 1 bis 5, dadurch gekennzeichnet, daß als Lösungsmittel bei der O-Substitution Aceton, Methylethylketon, Methanol, Ethanol, Wasser, Dimethylformamid, Dimethylsulfoxid oder deren Gemische verwendet werden.

7. Chromanonderivate der Formel I,

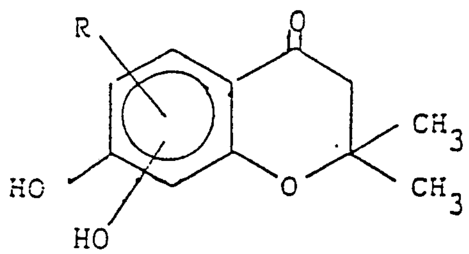

(I),

in der R Wasserstoff, Chlor oder $C_{1-8}$-Alkyl bedeutet, als Zwischenprodukte zur Herstellung entsprechender Chromenderivate.

**Revendications**

1. Procédé de préparation de dérivés de chromène de formule générale V

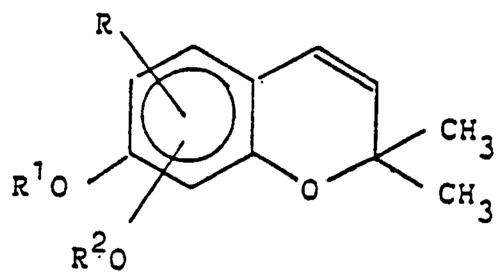

(V),

ou

R représente un hydrogène ou un alcoyle en $C_1$ à $C_8$ et

$R^1$ et $R^2$ représentent un alcoyle en $C_1$ à $C_{10}$, benzyle, trifluoroéthyle ou acétyle, où $R^1$ et $R^2$ sont différents, ou représentent ensemble un groupe $-(CH_2)_3-$,

caractérisé par
(a1) la réaction d'un dérivé de chromanone de formule générale I

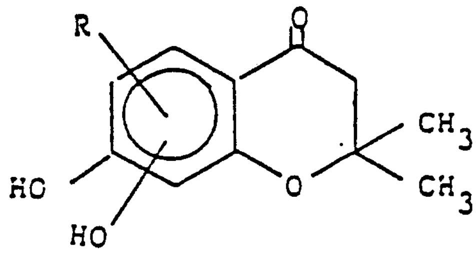

(I)

avec R comme ci-dessus
avec un réactif de formule générale

$R^1$-X,

où
$R^1$ est le même que ci-dessus et
X représente le radical d'un agent alcoylant ou acétylant,
dans une quantité approximativement équivalente pour donner un dérivé de chromanone mono-O-substitué de formule générale II

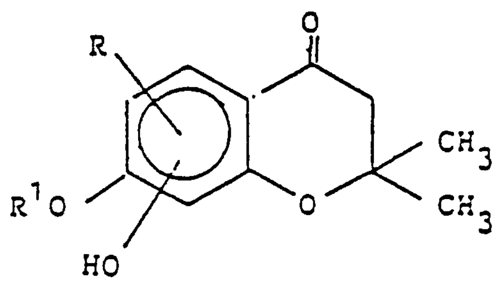

(II)

avec R et $R^1$ comme ci-dessus
et réaction du dérivé de chromanone de formule II avec un réactif de formule générale

$R^2$-X

avec $R^2$ et X comme ci-dessus en une quantité de 1,1 à 1,5 moles par mole du dérivé de chromanone de formule II pour donner un dérivé de chromanone di-O-substitué de formule générale III

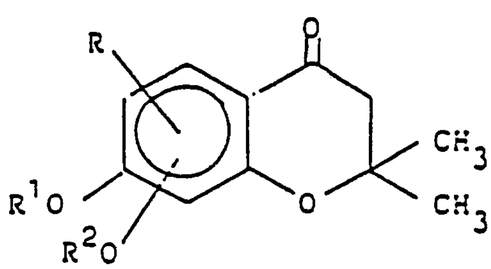

(III)

avec R, $R^1$ et $R^2$ comme ci-dessus
ou encore
(a2) la réaction directe du dérivé de chromanone de formule I avec du 1,3-dibromopropane pour donner le dérivé de chromanone de formule III où $R^1$ et $R^2$ représentent ensemble $-(CH_2)_3-$,
(b) la réduction en soi connue du dérivé de chromanone de formule III pour donner le chromanol correspondant de formule générale IV

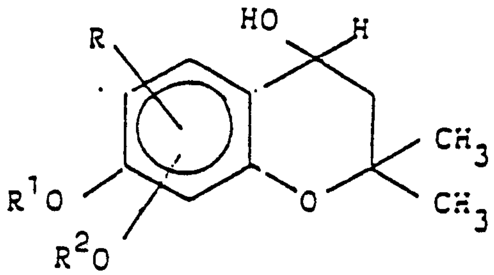

(IV)

avec R, $R^1$ et $R^2$ comme ci-dessus
et
(c) la déshydratation en soi connue du chromanol de formule IV dans un milieu acide aqueux.

2. Procédé selon la revendication 1, caractérisé en ce que dans la variante (a1) on utilise comme agents alcoylants des halogénures d'alcoyle, sulfates de dialcoyle ou tosylates de trifluoroéthyle correspondants.

3. Procédé selon les revendications 1 ou 2, caractérisé en ce que dans la variante (a1), on utilise comme agent alcoylant l'iodure de méthyle, l'iodure d'éthyle, l'iodure d'isopropyle ou le sulfate de diméthyle.

4. Procédé selon l'une des revendications 1 à 3, caractérisé en ce que pour la préparation des chromanones de formule générale II selon la variante (a1) on utilise le réactif de formule

$R^1$-X

avec $R^1$ et X comme ci-dessus en une quantité de 0,8 à 1,5 moles par mole du composé de formule I.

5. Procédé selon l'une des revendications 1 à 4, caractérisé en ce qu'on purifie les dérivés de chromanone mono-O-substitués de formule générale II sous la forme de leurs sels.

6. Procédé selon l'une des revendications 1 à 5, caractérisé en ce qu'on utilise comme solvant dans la O-substitution l'acétone, la méthyléthylcétone, le méthanol, l'éthanol, l'eau, le diméthylformamide, le diméthylsulfoxyde ou leurs mélanges.

7. Dérivés de chromanone de formule 5

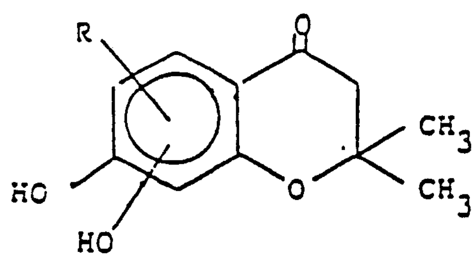

(I),

où R représente un oxygène, un chlore ou un alcoyle en $C_1$ à $C_8$,
comme produits intermédiaires pour la préparation de dérivés de chromène correspondants.

## Claims

1. Process for preparing chromene derivates of general formula V

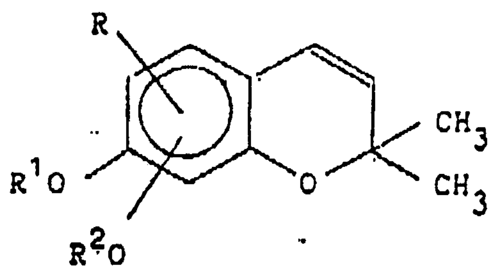

(V),

wherein
R represents hydrogen or $C_{1-8}$-alkyl and
$R^1$ and $R^2$ represents $C_{1-10}$-alkyl, benzyl, trifluoroethyl or acetyl,
$R^1$ and $R^2$ being different, or together they represent a group $-(CH_2)_3-$,

characterised in that
(a1) a chromanone derivative of general formula I

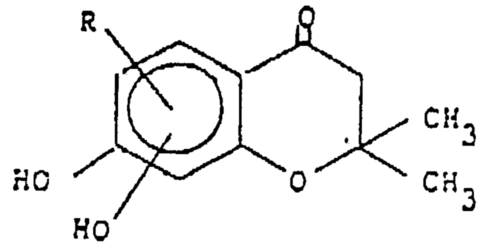

(I)

wherein R is defined as hereinbefore, is reacted with a reagent of general formula

$R^1$-X

wherein $R^1$ is defined as hereinbefore and
X represents the radical of an alkylating agent or acetylating agent,
in a substantially equivalent quantity, to obtain a mono-O-substituted chromanone derivative of general formula II

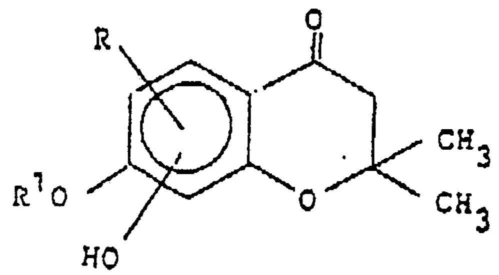

(II)

wherein R and $R^1$ are defined as hereinbefore and
the chromanone derivative of formula II is reacted with a reagent of general formula

$R^2$-X

wherein $R^2$ and X are defined as hereinbefore in a quantity of from 1.1 to 1.5 mol per mol of the chromanone derivative of formula II to obtain a di-O-substituted chromanone derivative of general formula III

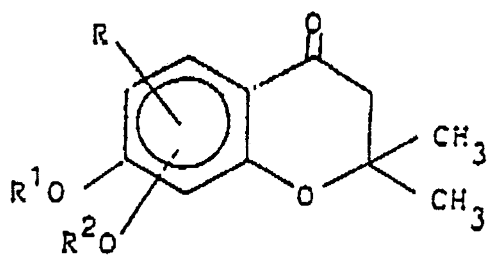

(III)

wherein R, $R^1$ arid $R^2$ are defined as hereinbefore
or alternatively
(a2) the chromanone derivative of formula I is reacted directly with 1,3-dibromopropane to obtain the chromanone derivative of formula III wherein $R^1$ and $R^2$ together represent $-(CH_2)_3-$,
(b) the chromanone derivative of formula III is reduced in a manner known <u>per se</u> to obtain the corresponding chromanol of general formula IV

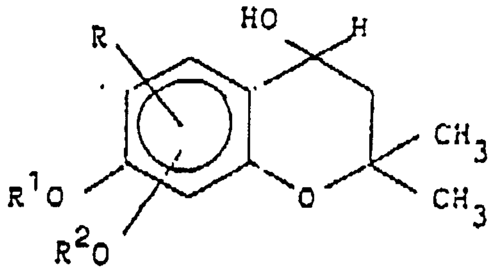

(IV)

wherein R, $R^1$ and $R^2$ are defined as hereinbefore
and
(c) the chromanol of formula IV is dehydrated in a manner known per se in an aqueous acidic medium.

2. Process according to claim 1, characterised in that in variant (a1) the alkylating agents used are corresponding alkylhalides, dialkylsulphates or trifluorethyltosylate.

3. Process as claimed in claim 1 or 2, characterised in that in variant (a1) the alkylating agent used is methyliodide, ethyliodide or isopropyliodide or dimethylsulphate.

4. Process as claimed in one of claims 1 to 3, characterised in that in order to prepare the chromanones of general formula II according to variant (a1) the reagent of formula

$R^1$-X

wherein $R^1$ and X are defined as hereinbefore is used in a quantity of from 0.8 to 1.5 mol per of the compound of formula I.

5. Process as claimed in one of the claims 1 to 4, characterised in that the mono-O-substituted chromanone derivatives of general formula II are purified in the form of their salts.

6. Process as claimed in one of claims 1 to 5, characterised in that the solvent used in the O-substitution is acetone, methylethylketone, methanol, ethanol, water, dimethylformamide, dimethylsulphoxide or a mixture thereof.

7. Chromanone derivatives of formula I,

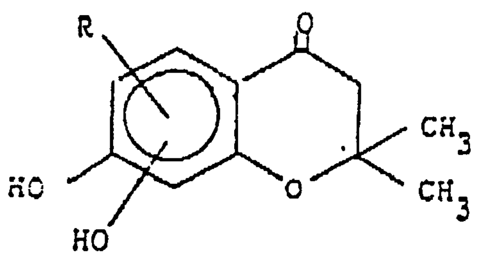

(I),

wherein R represents hydrogen, chlorine or $C_{1-8}$-alkyl,
as intermediate products for the preparation of corresponding chromene derivatives.